# EUROPÄISCHE PATENTSCHRIFT

(11) **EP 2 467 051 B1**
(45) Veröffentlichungstag und Bekanntmachung des Hinweises auf die Patenterteilung: **02.08.2017**
(21) Anmeldenummer: 10779680.7
(22) Anmeldetag: 17.08.2010
(51) Int. Cl.: A61B 1/00, G06T 3/40

(54) **BILDVERARBEITUNGSSYSTEM MIT EINER ZUSÄTZLICHEN ZUSAMMEN MIT DER BILDINFORMATION ZU VERARBEITENDEN MASSSTABSINFORMATION**
IMAGE PROCESSING SYSTEM HAVING AN ADDITIONAL PIECE OF SCALE INFORMATION TO BE PROCESSED TOGETHER WITH THE IMAGE INFORMATION
SYSTÈME DE TRAITEMENT D'IMAGE AVEC TRAITEMENT CONJOINT D'UNE INFORMATION D'ÉCHELLE ET DE L'INFORMATION D'IMAGE

(30) Priorität: 18.08.2009 DE 102009038021
(43) Veröffentlichungstag der Anmeldung: 27.06.2012
(73) Patentinhaber: Christiansen, Olaf, 14476 Potsdam (DE)
(72) Erfinder: CHRISTIANSEN, Olaf, 14476 Potsdam (DE); LÖHDE, Eckhard, 14129 Berlin (DE)
(86) Internationale Anmeldenummer: PCT/DE2010/050058
(87) Internationale Veröffentlichungsnummer: WO 2011/020471

(56) Entgegenhaltungen:
- US-A1- 2002 054 048
- US-B1- 6 891 148

## Beschreibung

Die Erfindung betrifft ein Bildverarbeitungssystem der im Oberbegriff des Anspruchs 1 angegebenen Art, das insbesondere für medizinische Zwecke verwendbar ist.

Derartige Bildverarbeitungssysteme werden heute beispielsweise in Form von digitalen Endoskop-Kameras sowohl in der allgemeinen Technik - bei schwer zugänglichen Reparaturstellen - als auch in der minimalinvasiven Chirurgie eingesetzt. Durch die kurze Brennweite der verwendeten Kameras weisen sie eine relativ große Schärfentiefe auf, welche auch notwendig ist, damit die Bedienungsperson einen guten Überblick über den Arbeitsbereich hat und die betrachteten Objekte nicht bei jeder Bewegung des Endoskops aus dem Schärfenbereich heraus gelangen. Die entsprechenden Kameras haben dabei eine feste Entfernungseinstellung, die dem Arbeitsbereich angepasst ist. Die Schärfentiefe kann bei bekannten Systemen beispielsweise einen Bereich von 1 mm bis Unendlich umfassen.

Da sich die zu betrachtenden Objekte eines Arbeitsbereichs damit - trotz scharfer Darstellung - in unterschiedlicher Entfernung von der Kameraoptik befinden können, kann von der dargestellte Größe auf dem Bildschirm nur schlecht auf die tatsächliche Größe eines Objekts geschlossen werden, zumal meist keine Vergleichsobjekte bekannter Größe vorhanden sind.

Das aus US 2002/0054048 A1 bekannte System zur Darstellung in tatsächlicher Größe mit einer digitalen Kamera und einem entsprechendes Display weist eine Messvorrichtung (z.B. Ultraschall-, Laserentfernungsmesser) für den Abstand zwischen Objekt und Bildebene auf, welche unter Einbeziehung der für den ermittelten Abstand hervorgerufenen Vergrößerung der Linse die Erzeugung eines Bildes in natürlicher Größe durch eine entsprechende Anpassung der Pixelgröße ermöglicht. Diese Lösung ist für die vorgesehene Anwendung ungeeignet, da ein zusätzliches Entfernungsmesssystem, wie es ja prinzipiell auch in der allgemeinen Phototechnik vielfach verwendet wird, für die Endoskopietechnik schon aus Raumgründen kaum anwendbar sein dürfte.

Aus der EP 1778084 B1 ist ein endoskopisches Video-Messsystem bekannt, mit dem Lichtpunkte im Bereich des Objekts erzeugt werden können, mit deren Hilfe im Bild Vermessungen durchgeführt werden können. Diese Vermessung erfolgt durch manuelle Auswahl von geeigneten Punkten und die daran anschließende Ermittlung des Bildmaßstabs durch eine im Bild vermessene Strecke. Anschließend erfolgt die Auswahl einer Bildeinzelheit, deren Abmessung bestimmt werden soll. Nachdem die betreffende Einzelheit im Bild manuell vermessen wurde, kann deren Länge dann aufgrund des Maßstabsfaktors errechnet und in der gewählten Maßeinheit angegeben werden. Soll ein von extern einzubringendes Element passend eingebracht werden, so ist auch dieses zu vermessen. Die Passgenauigkeit kann dann nur an Hand der beiden Maßzahlen beurteilt werden. Dieses Verfahren ist umständlich und für eine schnelle Handhabung ungeeignet.

Auch die US 2007/0065002 A1, in der ein System beschrieben ist, bei dem ein mit einem 3D-Laserscanner aufgenommenes Datenmodell mit 2D-Bildern aktualisiert werden kann, gibt insoweit keinen Hinweis.

Ferner sind aus DE 103 51 185 A1, US 4838245 A und US 2004/093906 A1 verschiedene konstruktive Maßnahmen für den Aufbau von Endoskopen mit Kameras und Beleuchtungsquellen und anderen Maßnahmen bekannt, die einen allgemeinen Überblick über die betreffende Technik geben.

In JAHNKE.M.: 3D-Exploration von Volumendaten, Diplomarbeit, Univ. Bonn , 1998, werden allgemein Werkzeuge zur nachträglichen interaktiven Erkundung von medizinischen Bilddaten beschrieben. Hinweise zur maßstabsgetreuen Wiedergabe bei interoperativer Anwendung finden sich nicht.

Im Vorlesungsskript VORNBERGER,O.,MÜLLER,O.: Computergrafik, Univ. Osnabrück, SS 2000, sind die Grundlagen des Gebietes der Computergrafik beschrieben, welche jedoch für die hier vorliegende Problemstellung keinen Hinweis liefern.

In JERAMIAS .R.F.: CMOS Bildsensoren mit Kurzzeitverschluss zur Tiefenerfassung nach dem Lichtlaufzeit-Messprinzip, ist ein Verfahren zur Entfernungsmessung nach dem Lichtlaufzeitprinzip beschrieben. Eine Entfernungsmessung ist aber im Zusammenhang mit dem vorstehend genannten Problem nicht unmittelbar von Bedeutung.

In GRIONI.C.: Digitalfotografie. Diplomarbeit Univ.Graz,2007, ist das Sony Smart-Zoom-Prinzip beschrieben, welches bei einem digitalen Zoom den Zoomfaktor entsprechend der gewählten Auflösung begrenzt, damit eine gewünschte Wiedergabequalität eingehalten werden kann. Dies ist aber im Zusammenhang mit dem hier beschriebenen Problem nicht relevant.

Der Erfindung liegt demgegenüber die Aufgabe zu Grunde, ein Bildverarbeitungssystem der eingangs genannten Art zu schaffen, mit dem es einer Bedienungsperson während einer endoskopischen Beobachtung auf einem Wiedergabebildschirm möglich ist, die Abmessungen eines einzubringenden Reparaturelements bzw. Implantats in real schnell zu ermitteln und unmittelbar für die Bearbeitung eines Werkstückes umzusetzen, ohne dass es dazu direkter Messungen im Inneren des endoskopisch betrachteten Raumes und nachträglicher Umrechnungen von Längenabmessungen bedürfte. Das gilt insbesondere auch für unter endoskopischer Beobachtung ausgeführte minimalinvasive Operationen, bei denen die Belastung des Patienten durch eine kurze OP-Zeit möglichst gering gehalten werden soll. (In der vorliegenden Beschreibung wird die Verwendbarkeit der Erfindung sowohl im medizinischen Bereich als auch im nichtmedizinischen Bereich durch die Gleichsetzung der Begriffe 'Arbeitsbereich' und 'Operationsbereich' einerseits und einzubringendes 'Element' bzw. 'Implantat' andererseits zum Ausdruck gebracht.)

Diese Aufgabe wird mit den im kennzeichnenden Teil des Anspruchs 1 angegebenen Maßnahmen gelöst. Es wird also auf einem Bildschirm das wiedergegebene Bild stets so eingestellt, dass es die aufgenommene Szene in einem festen Maßstab - bevorzugt 1:1- darstellt. Damit kann die Bildschirmfläche als Abbild der eigentlich unzugänglichen Szene direkt zu geometrischen Vergleichen herangezogen werden. Im einfachsten Fall bildet der Abstand einer Lasermarkierung von der optischen Achse die Bezugsgröße für den Maßstab.

Besonders vorteilhaft dabei ist, dass eine Größenabschätzung, Anpassung und Auswahl von Implantaten auch unmittelbar im sterilen Bereich des Operationssaals vorgenommen werden kann. Gerade bei minimalinvasiven Eingriffen kann die Anpassung des Implantats extern - ohne unmittelbaren und möglicherweise mehrfachen - Zugriff zum operierten Bereich vorgenommen werden. Bei modernen Operationstechniken im Bereich der Laparoskopie, insbesondere zur Behandlung von Leisten- und Zwerchfellbrüchen, kommt es auf die Einbringung von in ihrer Größe präzise angepassten Netzen an, was durch das hier dargestellte System auf einfache Weise und mit großer Genauigkeit möglich ist.

Durch die erfindungsgemäße Lösung ist es entweder möglich, unmittelbar auf einer durch den Bildschirm gebildeten Arbeitsoberfläche im Maßstab 1:1 mit direktem Vergleich von Werkstück und einzubringendem Element zu arbeiten oder aber in einem beliebigen Maßstab mit einer Abbildung des Werkstücks und einer Abbildung des einzubringenden Elements in entsprechendem Maßstab bzw. einer Teildarstellung im Maßstab 1:1. Damit lassen sich in der allgemeinen Technik auch größere Werkstücke oder Bereiche derselben bearbeiten, welche sonst die Abmessungen der Bildschirmfläche überschreiten würden. Es kann aber auch ein an eine Schadstelle angepasstes Modell in vergrößertem Maßstab manuell erstellt werden, das dann - beispielsweise mittels einer Kopierfräsmaschine - zur präzisen Herstellung des kleineren Originals dient.

Das erfindungsgemäße System eignet sich für alle Anwendungen, bei denen der Arbeitsbereich des Werkstücks für eine unmittelbare Anpassung nicht zugänglich ist. Dem Chirurgen bietet sich damit eine außerhalb des Operationsfeldes gelegene 'Werkbank', mit deren Hilfe er Anpassungen und Bearbeitungen vornehmen kann. Implantate und insbesondere Netze (meshes) für laparoskopische Hernienoperationen lassen sich durch Zuschneiden im Original passgenau vorbereiten oder auswählen. Die Auswahl kann entweder mit dem Original oder durch Einblenden einer Abbildung im entsprechenden Maßstab erfolgen. Dabei kann die Auswahl auch automatisiert erfolgen, wie es weiter unten beschrieben ist.

Bei der im Erfassungsbereich der optischen Digitalkamera aufgenommenen, für eine vorgegebene geometrische Distanz repräsentativen Information handelt es sich bevorzugt um das Zentrum mindestens einer von einem definiert und insbesondere parallel zur optischen Achse gerichteten Laserstrahlenbündel erzeugten Lasermarkierung als Projektion auf dem Objekt. Derartige Lasermarkierungen lassen sich durch kleine Einheiten erzeugen, welche ohne weiteres in oder in der Nähe der Optik eines Endoskops anbringbar sind. Es kann entweder der Abstand der Zentren zweier Lasermarkierungen untereinander oder der Abstand zwischen dem Zentrum einer Lasermarkierung und der optischen Achse der Kamera die für die geometrische Distanz repräsentative Information bilden.

Die Laserstrahlung kann aber auch zur optischen Achse geneigt sein, wenn dieser Abstand definiert in dem Sinne ist, dass für jeden Objektabstand der Ort des Auftreffens des Laserstrahlenbündels bestimmt ist, so dass nach Erfassung der Lasermarkierung eine geometrische Distanz als Bezugsentfernung mit Hilfe der Linsengleichung errechenbar ist. Unter Zuhilfenahme der optischen Achse, welche die Bildmitte bildet, sind bei einer definiert zur optischen Achse parallelen oder geneigten Führung des Laserstrahlenbündels für jeden ermittelten Auftreffpunkt der Abstand vom Objektiv und der Maßstab im Auftreffpunkt errechenbar.

Günstig ist es, wenn mehrere Laserstrahlenbündel um die optische Achse der Kamera herum angeordnet sind, wobei mehr als zwei Laserstrahlenbündel - bevorzugt gleichmäßig auf einem die optische Achse der Kamera umgebenden Kreisring - verteilt angeordnet sind. Dann sind nämlich für die Maßstabsermittlung diejenigen Lasermarkierungen ausschließlich auswählbar, welche definiert (insbesondere in gleichem Abstand zu ihren Nachbarn) angeordnet sind bzw. einer Symmetrie in Bezug auf die optische Achse folgen. Lasermarkierungen in davon abweichenden Positionen werden dann bei der weiteren Verarbeitung ausgeblendet oder in ihrem Gewicht bei der Bewertung verringert, wenn sie beispielsweise mit Vertiefungen oder Erhebungen zusammen fallen, die Schadstellen bzw. Verletzungen des Objekts bilden. Dabei ist es günstig, dass der Schnittpunkt der optischen Achse der Kamera mit dem Bild - also der Bildmittelpunkt auf dem Kamerasensor bzw. dem Bildschirm (welcher bei der Bearbeitung meist auf eine derartige Schadstelle zentriert werden wird) unabhängig von der Tiefe der Schadstelle stets einen korrekten Bezugswert liefert, weil die optische Achse als Bildmittelpunkt unabhängig von einer Abbildung auf dem Objekt definiert ist.

Auch wenn die Objektebene nicht senkrecht zur optischen Achse gerichtet ist, geben bei mehreren definiert zur optischen Achse ausgesendeten Laserstrahlenbündeln die einzelnen Lasermarkierungen auf dem Objekt (genauer gesagt: deren Abstand zur optischen Achse) ein Maß für den örtlichen Maßstab am Ort der Lasermarkierung. Ein unterschiedlicher Maßstab an zwei symmetrisch zur optischen Achse gelegenen Markierungen bedeutet, dass das Bild der Objektebene trapezförmig verzerrt ist (Keystone-Verzerrung). Durch eine entsprechende Entzerrung lässt sich somit eine wirklichkeitsgetreue Darstellung auf einem ebenen Bildschirm erzeugen, bei der die Neigung der Objektebene kompensiert ist. Im Allgemeinen lässt es sich - insbesondere mit einem flexiblen Endoskop - zwar erreichen, dass die die optische Achse der Kamera senkrecht auf die Objektebne gerichtet ist. Sollte dies jedoch einmal nicht der Fall sein, so kann mit den beschriebenen Maßnahmen Abhilfe geschaffen werden. Stehen mehr als zwei Quellen für Laserbündel zur Verfügung, so kann für die Ermittlung der Maßstabsfaktoren für die trapezförmige Verzerrung über die sich für die verschiedenen Laserpunkte ergebenden Maßstabsfaktoren gemittelt werden. Mit drei aufgenommenen Lasermarkierungen ist eine Ebene auch ohne Kenntnis von deren Neigungsrichtung definiert.

Vorteilhaft ist es auch, wenn zur Abtrennung der Lasermarkierung aus dem Bildinhalt die Bildinhalte zweier während eines Ein- oder Ausschaltvorganges der Laserlichtquellen unmittelbar aufeinanderfolgend aufgenommenen Bilder voneinander subtrahiert werden, so dass die Bildinhalte sich aufheben und die Lasermarkierungen zur Auswertung hervorgehoben sind. Um die Lasermarkierung im Erfassungszeitraum besser hervortreten zu lassen, sind zweckmäßigerweise die zur Objektbeleuchtung vorgesehenen Leuchtmittel, wie weiße LEDs, bei der Erfassung der Lasermarkierungen ausgeschaltet oder mindestens in ihrer Lichtstärke vermindert.

Eine Art 'Werkbank' ergibt sich für den Anwender, wenn das Bildverarbeitungssystem gemäß der Erfindung eine Auflagefläche zum unmittelbaren Aufbringen des Elements bzw. Implantats auf die im wesentlichen horizontal gerichtete Oberfläche des Bildschirms zwecks Größenabgleichs eines Teils des Arbeitsbereichs mit dem Element bzw. Implantat aufweist, wobei der Bildschirm selbst oder eine darauf befestigte Schutzscheibe als Auflagefläche dient und dabei unmittelbar die Arbeitsoberfläche.

Wenn der Bildschirm dabei in bevorzugter Weise als separate Einheit in einem getrennten Gehäuse mit autarker Stromversorgung und einer Funkschnittstelle zum übrigen System ausgestattet ist, kann die Wiedergabeeinheit ohne weiteres vom Ort der endoskopischen Bildaufnahme zum Ort der Anpassung des Reparaturelements oder Implantats mitgenommen werden.

Das erfindungsgemäße Bildverarbeitungssystem ist universell verwendbar und kann überall in der Medizin oder in der allgemeinen Technik eingesetzt werden, wo es darum geht, an unzugänglichen Arbeitsbereichen Reparaturen oder Modifikationen vorzunehmen. Dabei können entweder spezielle entsprechend vorbereitete Endoskope verwendet werden, bei denen Laserquellen an der Objektseite direkt in der Nähe der Kameraoptik angeordnet sind oder aber es können auch herkömmliche Endoskope verwendet werden, welche einen Arbeitskanal aufweisen, durch den dann das Laserstrahlenbündel mittels einer Faser geführt wird.

Wenn - entsprechend einer anderen vorteilhaften Ausführung der Erfindung Verschiebe- oder Rotationsmittel vorgesehen sind, mit deren Hilfe ein in das aufgenommene Bild eingeblendetes Abbild des einzubringenden Elements bzw. Implantats in seiner Position in der Darstellungsebene verschieb- oder drehbar ist, kann dessen geometrisches Zusammenwirken mit dem Werkstück bzw. dem Körper des Patienten im Arbeits- bzw. Operationsbereich in einfacher Weise überprüft werden. Andererseits können aber auch Tests und Versuche mit der Anpassung verschiedener Formen vorgenommen werden, welche auf diese Weise vor Ort nicht möglich wären.

Bei dem erfindungsgemäßen Bildverarbeitungssystem können weiterhin den Bildspeichermitteln nachgeschaltete Mittel zur hervorhebenden Auswahl von Bildelementen im Reparaturbereich vorgesehen sein, wobei die Auswahl nach vorgegebenen Kriterien aufgrund der aufgenommenen Bildinformation erfolgt. Die mit dem gewählten Merkmal versehene Bildinformation bildet eine 'Region of Interest (ROI)' die zur auswählenden Anpassung eines Reparaturelements verwendet werden kann. Der entsprechende Bereich kann aber auch zur Fernverarbeitung (Telemedizin) an eine entfernte Stelle übermittelt werden, um von dort eine Auswahl zu treffen.

Bei der die ROI bestimmenden Information kann es sich als Auswahlkriterium um die Farbe, die Intensität oder einen sonstigen gemeinsamen Bildinhalt handeln, wobei dann die Form oder Abmessungen dieses Bereichs bzw. der weitere Bildinhalt Kriterien für die automatisierte Auswahl des in den Arbeits- bzw. Operationsbereich einzubringenden Elements bzw. Implantats bilden können. In Erweiterung der bekannten Technik der element- oder inhaltsbasierten Bilderkennung, wird bei einer automatisierten Auswahl nicht nur ein Bild aufgrund seiner Inhaltsmerkmale aufgefunden, sondern aufgrund von Inhalten oder Elementen des Schadbildes ein Abgleich mit den Eigenschaften von zugeordneten geeigneten Elementen oder Inhaltsmerkmalen in einer Datenbank für Reparaturelemente bzw. Implantate getroffen werden, in denen diese nach Schadstellenmerkmalen klassifiziert und auswählbar sind. Nach der automatisierten Auswahl des Elements über einen Ähnlichkeitsabgleich der Anforderungsmerkmale mit den entsprechenden Merkmalen der zur Verfügung stehenden Reparaturelemente kann das geeignete körperlich aus einem Lager entnommen und auf den Bildschirm aufgelegt werden, um im 1:1-Maßstab gegebenenfalls noch zusätzlichen manuellen Anpassungen unterworfen werden.

Durch den mittels der betreffenden Kriterien hervorgerufenen Form, Farb- und sonstigen Merkmalsabgleich wird also aus einem in einem Speicher vorhandenen elektronischen Katalog, auf Grund der dort gespeicherten Abbildungen eine Auswahl eines geeigneten Elements bzw. Implantats unter genauer Angabe von Typ oder Bestellnummer, Größe etc. getroffen. Dabei erfolgt der Vergleich bevorzugt unter Verwendung einer besonderen Beschreibungssprache, welche die Kennzeichnung von Bildmerkmalen in komprimierter Form ermöglicht. Eine entsprechende Beschreibungssprache ist beispielsweise als MPEG7 definiert.

Bei der Anwendung im medizinischen Bereich handelt es sich insbesondere um laparoskopische Verwendungen, in der die Erfindung in günstiger Weise einsetzbar ist, weil hier eine Operationsplanung per Computer wegen der Beweglichkeit der inneren Organe des Menschen schwierig ist und viele Entscheidungen und Anpassungen unmittelbar in der Operationsphase getroffen werden müssen.

Die Anwendung der Erfindung ist nicht nur auf positive Formelemente beschränkt. Eine vorteilhafte Anwendung ist auch bei negativen Formen (Schablonen) möglich, wobei die Ausschnitte in der Schablone Arbeitsbereiche begrenzen oder aber Hinweiselemente in Form von aufgedruckten Zeichen enthalten können, welche die Arbeitsweise eines Chirurgen oder Feinmechanikers erleichtern.

Entsprechend sind weitere Elemente positiver Form günstig herstell- oder auswählbar, welche beispielsweise eine Haftvermittlung mit einer weiteren, später aufzutragenden Reparaturschicht erzeugen. Dabei kann es vorgesehen sein, dass sich die Schicht zur Haftvermittlung im Laufe der Zeit auflöst, oder auf einer Tragschicht ein- oder doppelseitig eine Kleberschicht aufweist.

Ein vorteilhaftes Ausführungsbeispiel der Erfindung ist in der Zeichnung näher dargestellt und wird nachfolgend näher beschrieben. Es zeigen:
Figur 1 das Prinzip des erfindungsgemäßen Systems in Blockdarstellung,
Figur 2 ein Diagramm mit verschiedenen Impulssignalen zur Erläuterung der Funktion des Systems gemäß Figur1,
Figur 3a eine perspektivische Darstellung eines ersten Ausführungsbeispiels eines Endoskops mit Kamera, welches mit den Maßnahmen gemäß der Erfindung versehen ist,
Figur 3b eine perspektivische Darstellung eines Messvorgangs mit einem Endoskop gemäß Figur 3a, bei dem die Objektebene nicht senkrecht zur optischen Achse gerichtet ist,
Figur 4 ein zweites Ausführungsbeispiel eines Endoskops in starrer Ausführung mit Kamera, welches ebenfalls mit den erfindungsgemäßen Maßnahmen versehen ist,
Figur 5 eine perspektivische Darstellung des Bildwiedergabeteils als Bedienungseinheit mit einem Bildschirm gemäß Figur 1 sowie
Figuren 6a und b je eine perspektivische Prinzipdarstellung zu weiteren Anwendungen der Erfindung.

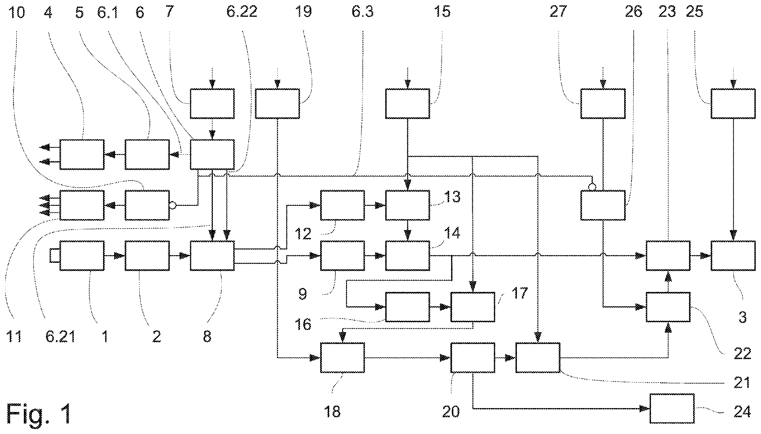

Bei dem in Figur 1 dargestellten Blockschaltbild des erfindungsgemäßen Bildverarbeitungssystems wird dessen Funktionsweise an Hand einer medizinischen Anwendung näher erläutert. Eine als Endoskopkamera ausgebildete Kamera 1 liefert ein Bild des Arbeitsbereichs. Das Ausgangssignal der Endoskopkamera 1 wird einem einen Puffer bildenden Bildzwischenspeicher 2 zugeführt, der jeweils das letzte Vollbild enthält, das beispielsweise im Progressive-Scan-Verfahren aufgenommen wurde. Damit steht für die nachfolgende Datenverarbeitung stets das letzte aktuelle Bild zur Verfügung, welches zur Darstellung auf einem Bildschirm 3 bereitgehalten wird. Bei fortlaufender Betrachtung ergibt sich eine bewegte Bildfolge.

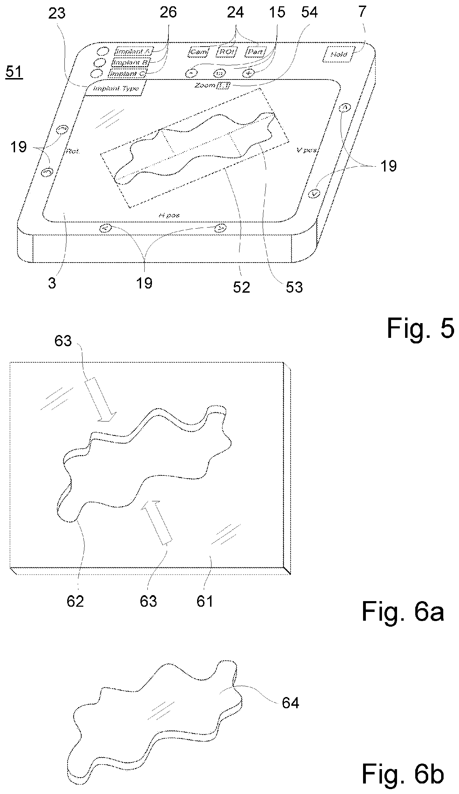

In der Nähe des Objektivs der Endoskopkamera 1 ist eine Laserlichtquelle 4 für ein oder mehrere parallel zur optischen Achse der Endoskopkamera 1 gerichtete Laserlichtbündel vorgesehen. (Die räumliche Anordnung ist weiter unten in Fig. 6 näher dargestellt.) Die Laserlichtbündel verlaufen parallel in einem festen Abstand zueinander. Sie erzeugen im Erfassungsbereich eine für eine vorgegebene geometrische Distanz auf dem Objekt repräsentative Information, die unabhängig von der Objektentfernung ist und zusammen mit der Bildinformation der Endoskopkamera 1 verarbeitet wird.

Anstelle eines Laserlichtbündels kann auch die optische Achse der Kamera als geometrische Referenz verwendet werden. Die Verarbeitung verläuft, unabhängig davon, ob als Distanzinformation zwei Lasermarkierungen oder eine Lasermarkierung und der Projektionspunkt der optischen Achse im aufgenommenen Kamerabild verwendet werden, im Wesentlichen gleichartig ab.

Auf dem Objekt, d.h. dem Arbeits- bzw. Operationsbereich entstehen beispielsweise zwei Lasermarkierungen, die im Kamerabild erscheinen und deren Abstand im Bild abhängig von der Entfernung zwischen Kamera und Objekt ist. Je nach Brennweite des Objektivs der Kamera 1 nimmt ihr Abstand auf dem Bild mit zunehmender Entfernung mehr oder weniger schnell ab und bildet so die Bezugsgröße für die Darstellung des Objekts auf dem Bildschirms 3 in einem vorgegebenen Maßstab. Entsprechend können auch mehrere Laserstrahlenbündel verwendet werden, die insbesondere die optische Achse der Kamera symmetrisch, und dabei insbesondere ringförmig, umgeben. (Diese Laserstrahlenbündel können auch einen definierten Winkel zur optischen Achse einnehmen, da damit aufgrund der geometrischen Beziehungen und der Linsenformel zu jeder erzeugten Lasermarkierung eine eindeutige Maßstabsinformation für den Bereich des Objekts gehört, auf dem die Lasermarkierung erscheint.)

Für den unmittelbaren Vergleich mit am Arbeitsort einzubringenden Elementen wird der Wiedergabemaßstab 1:1 gewählt, so dass Anpassungen unmittelbar am Bildschirm möglich sind. Für größere Objekte, die die Maße der Fläche des Bildschirms 3 überschreiten, lässt sich ein kleinerer Wiedergabemaßstab vorwählen, wobei in diesem Fall ein Abgleich nicht mit dem Originalobjekt, sondern lediglich mit einem gespeicherten verkleinerten Bild stattfindet. Um das einzubringende Element bearbeiten zu können, kann dann bedarfsweise für Teildarstellungen wieder auf den Maßstab 1:1 gewechselt werden, um die entsprechenden Teilbereiche des realen Elements zum Abgleich auf den Bildschirm als Arbeitsfläche auflegen zu können.

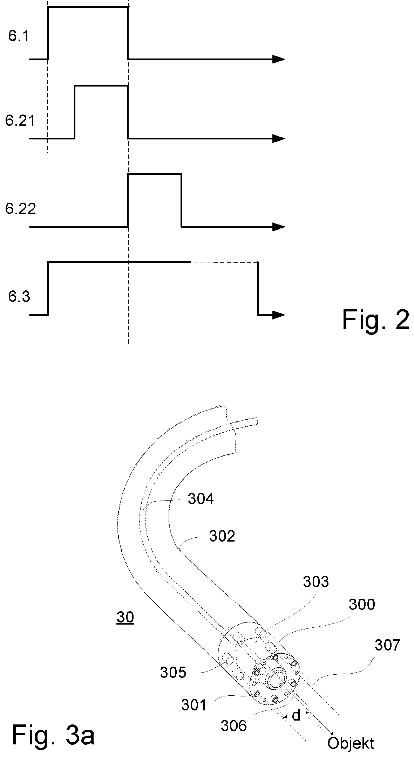

Die Laserlichtquelle 4 wird von einer Treiberstufe 5 gespeist, welche von einer Zeitgebereinheit 6 angesteuert wird. Die Zeitgebereinheit 6 liefert an ihren Ausgängen 6.1 und 6.2 Steuerimpulse, welche jeweils eine Sequenz bilden, die durch einen Auslöser 7 von Hand für einen Durchlauf aktiviert wird. Diese und die folgenden Impulssignale der Sequenz sind in Figur 2 im Einzelnen wiedergegeben, so dass insoweit jeweils auf diese Figur Bezug genommen wird. Der Auslöser 7 ist bistabil und liefert nach jeder zweiten bis zur darauf folgenden Betätigung ein 'Hold'-Signal 6.3. Dieses Signal dient dazu, das jeweils letzte von der Kamera 1 gelieferte und im Bildzwischenspeicher 2 festgehaltene Bildsignal in den nachgeschalteten Bildspeicher 9 zu überführen. Das Bild bleibt dort bis zur erneuten Betätigung des bistabilen Auslösers 7 als Standbild erhalten.

Bis zum Start der Puls-Sequenz bleibt das Bild der Kamera 1 zum Bildschirm 3 durchgeschaltet und bildet eine laufende Videosequenz mittels der die Kamera 1 auf das Objekt unter Betrachtung des Bildes ausgerichtet und in der Position kontrolliert werden kann. Der Auslöser 7 hat damit praktisch eine 'Festhalte-(Hold-) Funktion'. Er kann zweckmäßigerweise am Bedienteil des Endoskops angebracht bzw. mit dem Ausgangssignal des dort vorhandenen üblichen Auslösers zum Registrieren des Kamerabildes mittels einer ODER-Schaltung verknüpft sein, die hier nicht näher dargestellt ist.

Das Hold-Signal 6.3 des Auslösers gelangt zusätzlich invertiert an die Treiberstufe 10 für die Beleuchtung 11 des Objekts, welche aus einer Anzahl von weißen LEDs besteht. Diese Beleuchtung ist also während der normalen Wiedergabe der Videosequenzen eingeschaltet, so dass das Objekt beleuchtet ist, bis das letzte Bild der Videosequenz als Standbild festgehalten ist. Mit dem Erscheinen des Hold-Signals 6.3 verlischt die LED- Beleuchtung, so dass die Auswertung der Laserlichtsignale ohne störende Beleuchtung mit größter Genauigkeit erfolgen kann.

Die Sequenz der Impulse 6.1 bis 6.22 dient zur Steuerung der Auswertung der durch die Laserlichtquelle 4 hervorgerufenen Darstellung eines Bildes, welches verarbeitet und auf dem Bildschirm 3 dargestellt wird. In der Sequenz wird zunächst ein Bild der laufenden Darstellung durch eine Abtrenneinheit 8 bei abgeschalteten LEDs (Impuls 6.3) und eingeschalteter Laserlichtquelle (Impuls 6.1) festgehalten (Impuls 6.21). Unmittelbar nach Abschalten der Laserlichtquelle wird über den Impuls 6.22 in der Abtrennstufe 8 das nun erfasste Bildsignal in einem in der Abtrenneinheit 8 vom zuvor festgehaltenen Signal abgezogen. Da dies der unbeleuchtete Bildinhalt ist (LEDs und Laserquelle sind aus) verbleibt in diesem Speicher die durch die Laserquelle 4 hervorgerufene Lasermarkierung als Inhalt des Bildsignals. Das für eine genaue Auswertung maßgebliche Zentrum der Lasermarkierungen wird dabei geometrisch als deren Schwerpunkt ermittelt und/oder durch ein Intensitätskriterium, welches den Bereich größter Helligkeit der Lasermarkierung deren Zentrum zuordnet.

Die erhaltene Bildinformation gibt im Falle der Auswertung des Abbilds zweier Laserlichtbündel zwei Lasermarkierungen wieder, deren Zentren im von der Kamera aufgenommenen Bildinhalt einen Abstand aufweisen, welcher einer festen Distanz d auf dem Objekt entspricht. Die entsprechende Bildinformation wird in einen Abstandsauswertungsspeicher 12 übertragen und festgehalten. Die ermittelten Zentren der beiden Lasermarkierungen - also deren geometrische Mittelpunkte - werden dort in kartesischen Koordinatenwerten (abgeleitet von der Position der entsprechenden Pixel im Bildspeicher und gemäß Pythagoras aus der Distanz der jeweiligen Koordinatendifferenzen die Länge der Hypotenuse als Abstand in der Bildebene (gemessen in Pixelgrößen der Bildwiedergabe) ermittelt. Hinzu tritt ein systemspezifischer Multiplikator, der das Verhältnis der Größenabmessungen des physischen Bildschirms zur Pixelanzahl berücksichtigt, so dass im Falle des Maßstabs 1:1 die interessierenden Objekte des Arbeits- bzw. Operationsbereichs in natürlicher Größe erscheinen. Bei der Benutzung der optischen Achse der Kamera anstelle eines Laserstrahlenbündels wird anstelle des Mittelpunktes einer Lasermarkierung der Schnittpunkt dieser optischen Achse - also die Koordinaten des Bildmittelpunkts (0,0) ausgewertet.

Im Falle von mehreren, den Mittelpunkt in einem symmetrischen Muster umgebenden Lasermarkierungen, werden nur diejenigen ausgewertet, welche von diesem Muster, welches auf die optische Achse der Kamera - also die Bildmitte bezogen ist, um nicht mehr als einen vorgegebenen Betrag abweichen. Die Koordinaten-Informationen der verbleibenden Markierungen werden dann gemittelt und mindestens ein Distanzwert - bzw. die Mittelwerte mehrerer Distanzwerte zum Bildmittelpunkt zur Ermittlung des Maßstabsfaktors weitergegeben.

Um eine 1:1-Wiedergabe auf dem Bildschirm 3 zu erhalten, wird der erhaltene Distanzwert durch den realen geometrischen Abstand d der Laserlichtquellen 4 dividiert. Die entsprechende Berechnung erfolgt im Maßstabmultiplikatorteil 13. Das Ausgangsignal dieses Teils 13 gelangt zur Bildgrößeneinstelleinheit 14, welche eine elektronische Zoom-Funktion ermöglicht. Am Ausgang der Einheit 14 erscheint damit das normierte Bildsignal, welches auf dem Bildschirm zur Anzeige gelangt. Eine Bildschirmanzeige im 1:1-Maßstab ermöglicht damit eine direkte Beurteilung des Arbeitsbereichs im Standbild, so dass auf dem Bildschirm oder einer darauf angebrachten Glasplatte als Arbeitsunterlage alle Manipulationen in Bezug auf den Arbeitsbereich durchgeführt werden können, die am Originalarbeitsplatz auf Grund von dessen Unzulänglichkeit nicht möglich sind. Es können Zuschnitte und Formen angepasst und Elementen in direkter Anpassung an die Verhältnisse des Arbeitsbereichs vorgenommen werden, die sonst nicht möglich wären. Das gilt insbesondere für Reparaturelemente oder entsprechende Hilfsmittel.

Soll - beispielsweise zur besseren Übersicht - ein größerer Ausschnitt des Arbeitsplatzes wiedergegeben werden, so kann über einen zusätzlichen Schalter für die externe Maßstabseinstellung 15 der Wert 1:1 - entsprechend der Grundstellung des Schalters 15 - überfahren werden und damit die Darstellung des Arbeitsbereichs auf dem Bildschirm 3 in einem anderen Maßstab erzwungen werden. Das erfolgt dadurch, dass im Maßstabmultiplikatorteil 13 ein zusätzlicher Wert, der von der Maßstabseinstellung 15 stammt, einmultipliziert wird. Das hat aber nur Bedeutung, wenn von der 1:1 Wiedergabe abgewichen werden soll.

In dem System gemäß Figur 1 sind weiterhin Mittel dargestellt, welche es ermöglichen, aufgrund der Bilddarstellung - beispielsweise bei einem Schaden - ein Reparaturelement aufzufinden, welches eine dem Schadensereignis angemessene Größe und Form hat und auch in seinen sonstigen Eigenschaften an den Schadensfall angepasst ist. Im Falle einer laparoskopisch zu behandelnden medizinischen Hernienanwendung bedeutet das, ein Netz geeigneter Größe und Form auszuwählen bzw. herzustellen. Bei Reparaturen von unzugänglichen Behälterinnenräumen kann beispielsweise das Erfordernis bestehen, einen Reparatursatz geeigneter Größe zu finden, der mittels Laserschweißung aufgebracht werden kann. Weiterhin sind Anwendungen möglich, bei denen Schablonen zum definierten Umreißen eines Reparaturbereichs, Arbeitsvorlagen bzw. Schnittmuster oder Zwischenlagen zur Haftungsvermittlung von Reparaturmaterial größengetreu vorzubereiten sind. Diese Mittel werden weiter unten näher dargestellt.

Der Bildgrößeneinstelleinheit 14 ist ein Formdiskriminator 16 nachgeschaltet, der - ausgehend vom Zentrum der Bilddarstellung - benachbarte Pixelelemente innerhalb eines Fensters ähnlicher Eigenschaften (Intensität, Farbe, Musterungszusammenhang) diskriminiert und mit einer entsprechenden Kennzeichnung versieht. Anschließend wird ein Formglättungs- und Geschlossenheitskriterium angewandt, welches Pixelelemente, die sich innerhalb einer geschlossenen für einen zu behandelnden Defekt typischen Form befinden. Der Formdiskriminator ist beispielsweise geeignet, Bereiche mit gleichartigen Färbungen auszuwählen und als geschlossenen Formbereich darzustellen. Dieser Formbereich kann dann in seiner Darstellung auf dem Bildschirm als Basis zur Herstellung einer Schablone oder einem Implantat dienen. Zum anderen kann die Form aber auch 'virtuell' weiterverarbeitet werden und als Basis für die Auswahl eines geeigneten Reparaturelements aus einem Katalog dienen.

Das so gewonnene zu einem einheitlichen geschlossenen Gebiet hervorgehobene Gebilde wird einer Anpassung in der Größe in einer Größenkorrektureinheit 17 entsprechend dem mit dem Einsteller 15 eingestellten Maßstab unterworfen (falls dieser nicht 1:1 ist), so dass die ermittelte Form der Größe der Reparaturstelle entspricht - auch wenn diese über die Möglichkeiten des Bildschirms 3 in der 1:1-Darstellung hinausgeht. Dieser Bereich wird ROI (Region of Interest) genannt und gibt in schematischer Form den Defektbereich graphisch wieder, so dass auf Grund dieser Daten ein Formvergleich mit in einem nach Art eines Kataloges organisierten Speicher erfolgen kann, in dem die für das betreffende Anwendungsgebiet in Betracht kommenden Elemente nach Typ mit Typenbezeichnung und Abbildungen festgehalten sind.

In einer nachgeschalteten Formvergleichseinheit 18 kann ein Reparaturelement von prinzipiell geeigneter Form aus einem vorgegebenen Bestand herausgesucht werden. Für den Formenvergleich sind verschiedene mathematische Verfahren geläufig, wie beispielsweise die Erkennung der Formähnlichkeit durch Minimierung von zwischen den Formen eingeschlossener Rechtecke. Dies würde in einer Pixeldarstellung erfolgen. Bei dem dargestellten Ausführungsbeispiel soll aber davon ausgegangen werden, dass der Vergleich der Grafikelemente in einer komprimierten Form unter Benutzung von Auswahlkriterien und Methoden der Formkennzeichnung erfolgt, wie sie beispielsweise in MPEG7 standardisiert sind. Das Auswahlverfahren verwendet Verfahren der element- oder inhaltsbasierten Bilderkennung in der Weise, dass dem Bild der Schadstelle oder Verletzung bestimmte Form- und Farb- und Maßelemente zugeordnet werden, die für den jeweiligen Anwendungsfall charakteristisch sind. Den Repararaturelementen bzw. Implantaten sind diese Elemente als Auswahlkriterien zugeordnet. Auf diese Weise ist durch das Auffinden bestimmter inhaltlicher Elemente des Bildes der Schadstelle unmittelbar visuell und automatisch das geeignete Reparaturelement auswählbar.

Über eine manuelle Auswahlfunktion, mit der über einen Auswahlschalter 19 zusätzliche Formauswahlkriterien oder zusätzliche Selektionsmerkmale für das auszuwählende Element manuell hinzufügbar sind, wird ein Selektionssignal mit der gesamten Forminformation einem Elementenkatalogspeicher 20 als Eingangssignal zugeführt. In diesem Elementenkatalogspeicher 20 sind alle für die Anwendung in Betracht kommenden Elemente mit einer Bilddarstellung verzeichnet.

Dabei sind zu jedem Element mindestens zwei Abbildungen gespeichert, von denen jeweils eine realistische Darstellung als eine Art Fotowiedergabe (beispielsweise in Form von JPEG-Daten) bildet, während die andere schematisierender (also Bildinhaltsbezogener) Art ist und zu Vergleichszwecken bei der Selektion dient. Hierbei sind bevorzugt lediglich Formmerkmale gespeichert, die mit entsprechenden ausgewählten Formmerkmalen der Schadstelle verglichen werden können. Hierbei erfolgt eine Kodierung entsprechend der Norm MPEG7, so dass bei dem Formvergleich lediglich die einzelnen Merkmale als Digitalwerte verglichen werden müssen. Dabei ist Voraussetzung, dass bei der Formdiskriminierung im Formdiskriminator zusätzlich eine Datenkompression in der Weise erfolgt, dass nicht die Pixelinformation gespeichert wird, sondern deren Beschreibung mit den durch das Kompressionsverfahren vorgegebenen 'Datenworten', welche dann mit den entsprechenden das Reparaturelement beschreibenden Datenworten verglichen werden. Damit vereinfacht sich der Formvergleich in der Einheit 18 wesentlich. Auch die Größenkorrektur in der Einheit 17 beschränkt sich dann nur noch auf eine Veränderung von Größenbezeichnungen, so dass die Elemente in dem Katalogspeicher nur noch jeweils in einer allgemeinen Formabbildung abgespeichert sein müssen.

Durch das Selektionssignal wird das geeignete Element in Bilddarstellung ausgewählt und über einen Zwischenspeicher 21, eine Schalteinheit 22 und eine Überlagerungseinheit 23 dem aktuellen im Bildschirm 3 dargestellten Bild überlagernd hinzugefügt, wobei der auf dem Bildschirm 3 dargestellte Arbeitsbereich durch das Bild des zugefügten Elements überdeckt wird.

Um sicherzustellen, dass die Wiedergabe des ausgewählten Elements in seinen Abmessungen auch dem Maßstab des Arbeitsbereichs entspricht, wird die vom Elementenkatalogspeicher 20 an den Bildschirm 3 ausgegebene Bildinformation entsprechend dem an der externen Einstellung 15 eingestellten Maßstab skaliert.

Zur Information der Bedienungsperson wird vom Elementenkatalogspeicher 20 die Typenbezeichnung des ausgewählten Elements zu einer entsprechenden Anzeigeeinheit für den Elemententyp 24 ausgegeben, welche bei der praktischen Ausführung in Bildschirm 3 eingeblendet wird, wie es weiter unten näher dargestellt ist.

Zur Verdeutlichung sei hier noch einmal das Prinzip der automatisierten Auswahl eines Reparaturelements (Implantats) entsprechend der Blockdarstellung beschrieben, welches nicht an eine endoskopische Anwendung gebunden ist.
1. Auswahl des Reparaturbereiches aufgrund einer Selektion entsprechender Bildmerkmale in einem zusammenhängenden Gebiet (ROI).
2. Inhaltsbasierte Analyse zum Auffinden charakteristischer (visueller) Bildmerkmale innerhalb der ROI, dies beinhaltet deren Abmessungen entsprechend der Charakterisierung von Bilddateien zur Vorbereitung auf die inhaltsbasierte Bildsuche.
3. Zusammenfassung der Bildmerkmale der für die Reparaturstelle relevanten Bildmerkmale zu Anforderungseigenschaften für die Reparaturelementauswahl in der Datenbank.
4. Umformung der zusammengefassten Bildmerkmale in Auswahlkriterien zur Adressierung der Datenbank, in der die Elemente nach diesen Kriterien geordnet sind.
5. Auslesen der Typenbezeichnung mit Bilddatei und Übertragung der Bilddatei in den Bildschirm.

Die Darstellung im Bildschirm 3 kann mittels eines Darstellungsauswahlschalters 25 umgeschaltet werden, zwischen der reinen Darstellung des Arbeitsbereichs einerseits und der Überlagerung des aufgenommenen Bildes mit der selektierten ROI (Region of Interest) bzw. des ausgewählten Reparaturelements bzw. Implantats anderseits. Diese Darstellungsauswahl und -überlagerung wird durch ein UND-Gatter 26 mit einem invertierenden Eingang unterbunden, wenn auf dem Bildschirm 3 durch den Zeitgeber 6 die Videofunktion aktiviert ist. Das entsprechende Signal am invertierenden Eingang des UND-Gatters 26 stammt von der Zeitgebereinheit 6 und schaltet auch die Beleuchtung 11 aus.

Mittels eines Positionsschalters 27 lässt sich auf dem Bildschirm die Darstellung des Elements gegenüber dem Arbeitsbereich verschieben, um eine Optimierung der Anpassung zu ermöglichen.

Wird über die Maßstabseinstellung 15 von einer maßstäblich verkleinerten Wiedergabe auf den Maßstab 1:1 umgeschaltet, so wird das Element nur teilweise wiedergegeben, wenn seine Abmessungen über diejenigen des Bildschirms 3 hinausgehen. In diesem Fall wird ein Abgleich nur für den Teilbereich vorgenommen.

Bei der in Figur 3a wiedergegebenen Detaildarstellung des Kamerakopfes 300 eines Endoskops 30 sind am Ende der flexiblen Endoskophülse 302 eine Kamera 303 mit fester Brennweite (als Bildaufnahmemittel 1 gemäß Figur 1) und mehrere um die optische Achse 306 herum angeordnete zu dieser parallele aus Quellen 305 stammende Laserlichtbündel 305 in einem vorgegebenen Abstand d zur optischen Achse 306 montiert. Die elektrischen Zuleitungen sind dabei in einem Kabel 304 geführt, das im Inneren der flexiblen Endoskophülse 302 verläuft. Weiterhin sind um das Gehäuse der Kamera 503 herum zum Objekt hin strahlende weiße LEDs 301 vorgesehen, welche das Objekt beleuchten und ebenfalls über das Kabel 304 mit Strom versorgt werden.

Auf dem Objekt bilden die Laserlichtbündel 307 Punkte, deren Abstand d auf dem Objekt unabhängig vom Abstand des Kameraobjektives zum Objekt ist, da sie parallel gerichtet sind und einen im wesentlichen konstanten Querschnitt aufweisen. Die auf dem Objekt entstehenden Lasermarkierungen werden in Abhängigkeit von ihrer Übereinstimmung des Abstands zur optischen Achse 306 ausgewählt, weil dann sichergestellt ist, dass das Objekt in den markierten Bereichen einen übereinstimmenden Abstand zum Kameraobjektiv aufweist, welche für den Arbeitsbereich kennzeichnend ist. In ihrem Abstand von der optischen Achse im Bild abweichende Lasermarkierungen werden bei der Mittelung zur Erzeugung des Distanzwertes für die Berechnung des Maßstabsfaktors außer Betracht gelassen.

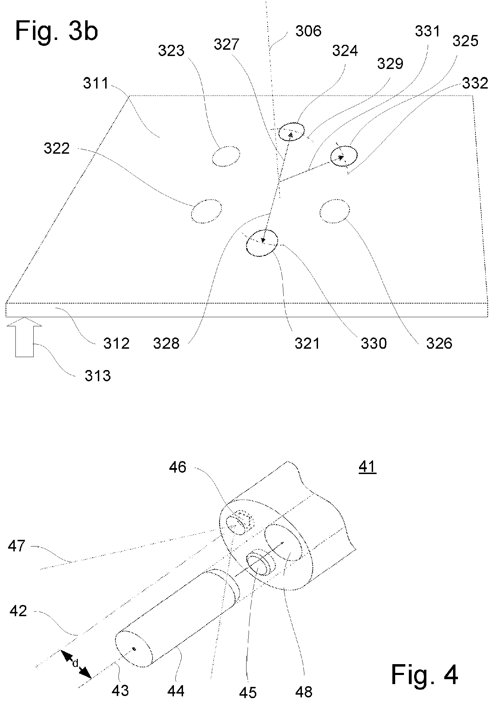

Bei der in Figur 3b wiedergegebenen Prinzipdarstellung ist verdeutlicht, wie mit einem Endoskop und den beschriebenen Schaltungsmitteln auch eine Ebene im Maßstab 1:1 wieder gegeben werden kann, welche nicht lotrecht zur optischen Achse 306 der in das Endoskop 30 (Figur 3a) eingebauten Kamera gerichtet ist. Die Kamera 303 ist auf eine in Figur 3b wiedergegebene quadratische Blechplatte 311 gerichtet, welche in Bezug auf die Papierebene an der dem Betrachter zugewandten Kante 312 in Richtung des Pfeils 313 angehoben ist, so dass sie hier einen Abstand zu einer Ebene aufweist, die senkrecht zur optischen Achse 306 gerichtet ist. Die sechs Laserlichtquellen 301 (Figur 3a) erzeugen auf der Platte 311 (Figur 3b) sechs Lasermarkierungen 321bis 326 in Form von kleinen Kreisscheiben. Für die Art der in der Figur dargestellten Neigung ist an sich die Auswahl von zwei Lasermarkierungen 321 und 324 hinreichend, da diese genau in Richtung der Neigung liegen. Die Lasermarkierung 321 hat dabei den größten und die Lasermarkierung 324 den kleinsten Abstand (Pfeil 327 bzw. 328) von der optischen Achse 306, was durch die gestrichelten teilkreisfömigen Hilfslinien 329 und 330 verdeutlicht ist. Um die Bilddarstellung zu entzerren und eine getreue 1:1- Wiedergabe auf dem Bildschirm zu ermöglichen, erfolgt eine Trapezentzerrung der Abbildung in der Weise, dass beide Lasermarkierungen gleiche Abstände von der optischen Achse erhalten (Block 14 in Figur 1). Die Anordnung der Lasermarkierungen folgt der trapezförmigen Verzeichnung der quadratischen Platte 311, da deren Auswanderung ein Maß für den örtlichen Maßstab ist und die Platte selbst ihre Maße in der Bildwiedergabe entsprechend verändert. Haben die beiden Lasermarkierungen 321 und 324 wieder den gleichen Abstand von der optischen Achse 306, so ist die Wiedergabe der Platte entzerrt und sie erscheint auf dem Bildschirm quadratisch. Bei parallel gerichteten Laserstrahlbündeln entspricht die Anordnung der Lasermarkierungen der Anordnung der Laserquellen 301 in Figur 3a. Die Abbildung hat also insgesamt den Maßstab 1:1, wenn die Verteilung (Abstände) der Lasermarkierungen 321 bis 326 den Maßverhältnissen an der Quelle entspricht. Sind die ausgewählten Lasermarkierungen nicht in Richtung der Neigung angeordnet, wie zuvor beschrieben, genügen im allgemeinen Fall deren drei, für die Wiedergabe einer zur optischen Achse nicht orthogonal gerichteten Objektebene. Dazu wird beispielsweise die Lasermarkierung 325 zusätzlich zur Auswertung herangezogen. Ihr Abstand 331 (Kreis 332) zur optischen Achse 306 liegt zwischen den Werten der Abstände 327 und 328 und das trifft auch auf den örtlichen Maßstabsfaktor zu. Auch bei der Verwendung von drei Lasermarkierungen erfolgt die Keystone-Entzerrung der Abbildung so, dass alle drei Abstände dem Originalabstand der Laserlichtquellen 301 von der optischen Achse 306 entsprechen. Damit kann die Platte 311 in Bezug auf die Senkrechte zur optischen Achse 306 beliebig geneigt sein. Die übrigen Lasermarkierungen 322, 323 und 326 wurden bei der vorstehenden Bildentzerrung nicht benutzt. Es besteht aber die Möglichkeit, die Entzerrung der Wiedergabe durch Benutzung aller Lasermarkierungen durch Mittelung ihres Einflusses zu optimieren oder es werden solche Lasermarkierungen, welche von den bei der Neigung einer ebenen Platte zu erwartenden Abständen deutlich abweichen, von der Weiterverarbeitung ausgeschlossen.

Figur 4 stellt ein zweites Ausführungsbeispiel der Erfindung in der Anwendung auf ein Endoskop 40 in starrer Ausführung mit Kamera dar, welches ebenfalls mit den erfindungsgemäßen Maßnahmen versehen ist. Das Endoskop 41 entspricht in seinen grundsätzlichen Funktionen denjenigen gemäß Figur 3, bildet dabei aber ein Standardendoskop, das in keiner Weise für die Anwendung mit der Erfindung vorbereitet zu sein braucht. Es sind beispielweise keine besonderen Optiken zur Erzeugung von Laserlicht erforderlich. Die dazu erforderlichen Maßnahmen werden durch zeitweises Einbringen in den Arbeitskanal des eingesetzten Endoskops getroffen. Dabei kann das Einsetzen der Laserquelle während des Einsatzes des Endoskops von der dem Bediener zugewandten Seite her erfolgen. Da der Arbeitskanal eine präzise Führung bildet, ist eine genaue Ausrichtung des Laserstrahlenbündels parallel zur optischen Achse ohne besonderen Aufwand gewährleistet.

In Figur 4 bildet die optische Achse 42 einer digitalen Miniaturkamera 46 mit dem Bildwinkel 47 eine Bezugslinie, wobei die Distanz D den Abstand zwischen der optischen Achse 42 und dem parallel dazu gerichteten Laserlichtbündel 43 bildet, welches aus der Laserlichtquelle 44 stammt, die ein zylindrisches Gehäuse aufweist, das in seinen äußeren Abmessungen an die inneren Abmessung, d.h. den Innenquerschnitt des Arbeitskanals 48 angepasst ist. Da der Arbeitskanal 48 einen gleichmäßigen Querschnitt aufweist, bildet er eine Führung für das Gehäuse der Laserlichtquelle 44, die in jeder beliebigen Höhe innerhalb des Arbeitskanals gebrauchsfertig ist. Damit kann die Laserlichtquelle in Koordination zu den anderen notwendigen Tätigkeiten bei Bedarf zeitweise in den Arbeitskanal von der Bedienerseite eingeführt werden, wenn andere Werkzeuge nicht zugegen sind. Mittels der erfindungsgemäßen Maßnahmen erfolgt dann die Vermessung, Inspektion oder sonstige Behandlung des Arbeitsbereiches, um letztlich ein Reparatur- oder sonstiges an die Schadensstelle angepasstes Hilfselement zu erhalten, welches dazu beiträgt, das Arbeitsziel zu erreichen. Außerdem ist an der Arbeitsseite des Endoskops 41 eine durch weiße LEDs gebildete Lichtquelle 45 vorgesehen, welche zur Beleuchtung des Arbeitsbereichs dient.

Bei der in Figur 5 wiedergegebenen Detaildarstellung des Bildwiedergabeteils des Systems gemäß Figur 1 kommt ein sogenannter Slate-Tablet-PC 51 zur Anwendung, der ein Touchscreen-Display und eine entsprechende Programmierung aufweist. (Die Programmierung umfasst auch den Umfang der in Figur 1 dargestellten Funktionen, wobei die externen Elemente, wie Kamera und Lichtquellen über entsprechende USB-Schnittstellen gesteuert werden können.) Das Display des Tablet-PC 51 bildet dabei die gesamte obere Deckfläche des Geräts, wobei die dargestellten Bedienungselemente in das wiedergegebene Bild eingeblendet sind und durch Berühren bedient werden können. Der zur Wiedergabe der Information des Bildschirms gemäß Figur 1 dienende Teil 3 füllt damit also nur einen Teil des Displays des Tablet-PC 51 aus. Auf diesem Bildschirmteil 3 ist als Beispiel für eine Anwendung ein rechteckiges Reparaturelement 52 in Bezug auf einen Schadensbereich 53 des betreffenden Arbeitsbereichs dargestellt. Weiterhin sind verschiedene Betätigungselemente gezeigt, deren Funktion der Beschreibung in Figur 1 entspricht. Sie lassen sich durch Berühren betätigen. Es handelt sich dabei um die folgenden Elemente: Auslöser 7, Maßstabeinstellung 15, Überlagerungseinheit 23, Darstellungsauswahlschalter 25 und Positionsschalter 27. Weiterhin ist im Bildschirmbereich eine die Funktion der Elemententyp-Anzeigeeinheit 24 realisiert, welche den Typ eines - wie zuvor beschrieben - aus dem Elementenkatalogspeicher 20 gemäß Figur 1 ausgewähltes Element 52 dem Typ nach anzeigt. Bei dem Element 52 kann es sich beispielsweise um ein chirurgisches Netz handeln, welches zur Abdeckung einer den Schadensbereich 52 bildenden Hernie dient. Bei beliebiger Maßstabstabseinstellung kann der Abgleich virtuell erfolgen, wobei über den Schalter 25 noch eine zusätzliche Typauswahl (beispielsweise nach Hersteller oder Dicke) getroffen werden kann. Über den Positionsschalter 27 kann die Darstellung des Elements 52 horizontal oder vertikal verschoben werden, um die Anpassung zu optimieren oder zu kontrollieren. Dazu kann zusätzlich über den Auswahlschalter 25 die getroffene Auswahl durch Darstellung der zur Auswahl herangezogenen ROI - des als auf Grund von Bildinhaltskriterien selektierten Bereichs in der Bilddarstellung des Arbeitsbereichs - kontrolliert werden.

Im Falle der Auswahl einer Darstellung im Maßstab 1:1 über die Maßstabeinstellung 15 kann ein Abgleich mit einem Originalelement vorgenommen werden, das auf die Deckfläche des Tablet-PC aufgelegt wird. Dabei kann ein individueller Zuschnitt erfolgen, ohne dass es eines direkten Zugriffs zum Operationsbereich bedarf, so dass eine entsprechende Belastung des Patienten vermieden ist. Der PC 51 bildet bei einer alternativen Ausführungsform einen Bildschirm, der eine autarke Stromversorgung aufweist und über eine Funkschnittstelle mit dem übrigen System verbunden ist. Auf diese Weise kann der Bildschirm weitergereicht und dort benutzt werden, wo die Form-anpassung eines Werkstücks erfolgen soll.

Bei den in den Figuren 6a und b wiedergegebenen perspektivischen Prinzipdarstellungen zu einer weiteren Anwendungsform der Erfindung, gibt Figur 6a eine Schablone 61 wieder, welche dazu benutzt werden kann, einen Bereich eines unzugänglichen Arbeitsbereichs von einem Arbeitsvorgang auszuschließen. Die Schablone kann extern mittels der erfindungsgemäßen Maßnahmen hergestellt werden, wobei beispielsweise ein Abgleich der zu erzeugenden Öffnung 62 mit einem Schadensbereich erfolgt, der bei einer nachfolgenden Bearbeitung ausschließlich erreicht werden soll. Das kann beispielsweise bei Reparaturen durch Materialauftrag mittels Laserschweißung der Fall sein. Zusätzlich können Markierungen 63 vorgesehen sein, welche eine genaue Orientierung erlauben und insbesondere bei Arbeiten mittels Endoskop hilfreich sind, da hier gegebenenfalls die Übersichtlichkeit eingeschränkt ist. In diesem wird die aufgerollte Schablone über den Arbeitskanal des Endoskops zugeführt und vor Ort entrollt.

In Figur 6b ist entsprechend ein Element 64 von positiver Form dargestellt, welches eine Haftungsvermittlung ermöglicht. Dabei kann es sich beispielsweise um einen chirurgischen Kleber auf einer Tragschicht handeln.

## Patentansprüche

1. Bildverarbeitungssystem zur Wiedergabe eines Bildinhalts in einem vorgegebenen Maßstab enthaltend
- eine optische Kamera großer Schärfentiefe mit einem digitalen Bildsensor sowie mit Mitteln, welche im Erfassungsbereich der Kamera eine für eine vorgegebene geometrische Distanz innerhalb des Bildes repräsentative Information als mit einer Laserlichtquelle projiziertes Muster erzeugen, die zusammen mit der Bildinformation von der Kamera aufgenommen und verarbeitet wird,
- Bildspeichermittel (9) und einen Bildschirm (3) zur Wiedergabe der aufgenommen Bildinformation, wobei
- der Kamera (1) nachgeschaltete Abtrennmittel (8), welche die für die vorgegebene geometrische Distanz repräsentative Information von der Bildinformation separieren,
- den Abtrennmitteln (8) nachgeschaltete Auswertungsmittel (13), welche aufgrund der für eine vorgegebene geometrische Distanz repräsentativen Information einen physikalischen Distanzwert ermitteln und ausgeben,
- Stellmittel (14) zur Veränderung der auf dem Bildschirm (3) wiedergegebenen Bildgröße nach Art eines digitalen Zooms, für die der physikalische Distanzwert eine Eingangsgröße bildet, zur Einstellung dieser Bildgröße entsprechend dem vorgegeben Maßstabsfaktor, in den der physikalische Distanzwert als Multiplikator einbezogen ist, derart dass ein im Bildinhalt enthaltenes Objekt, dessen geometrische Abmessung der geometrischen Distanz entspricht, auf dem Bildschirm mit einer Abmessung wiedergegeben wird, die mit dem vorgegebenen Maßstabsfaktor multipliziert ist.

2. Bildverarbeitungssystem nach Anspruch 1, **dadurch gekennzeichnet, dass** es sich bei der im Erfassungsbereich der Kamera (1) aufgenommen, für eine vorgegebene geometrische Distanz repräsentativen Information um mindestens eine jeweils von einer mindestens definiert zur optischen Achse der Kamera (1) gerichtete Strahlenbündel aussendenden Laserquelle (4) erzeugte Lasermarkierung handelt, wobei der Abstand zweier Lasermarkierungen untereinander bzw. der Abstand zwischen einer Lasermarkierung und der optischen Achse der Kamera (1) die für die geometrische Distanz repräsentative Information bildet.

3. Bildverarbeitungssystem nach Anspruch 1, **dadurch gekennzeichnet, dass** bei mehreren Lasermarkierungen, die um die optische Achse der Kamera (1) herum angeordnet sind, Mittel vorgesehen sind, welche diejenigen Lasermarkierungen von der Weiterverarbeitung ausschließen, die von einer auf die optische Achse der Kamera bezogenen Symmetrieposition um mehr als einen vorgegebenen Betrag abweichen.

4. Bildverarbeitungssystem nach Anspruch 1, **dadurch gekennzeichnet, dass** im Falle einer Objektebene, die nicht senkrecht zur optischen Achse der Kamera ausgerichtet ist, Mittel vorgesehen sind, um die Bildgröße auf dem Bildschirm mit einem lokal veränderlichen Maßstabsfaktor - entsprechend einer Trapez- oder Keystone-Verzerrung - derart einzustellen, dass sie zwischen mindestens zwei für eine geometrische Distanz repräsentativen Informationen und den sich daraus ergebenden Maßstabsfaktoren für die lokal einzustellende Bildgröße maßgeblich ist, interpoliert und gegebenenfalls außerhalb der Lasermarkierungen entsprechend extrapoliert ist.

5. Bildverarbeitungssystem nach Anspruch 1, **dadurch gekennzeichnet, dass** die Abtrennmittel (8) derart ausgebildet sind, dass aus dem Bildinhalt zwei während eines Ein- oder Ausschaltvorganges der Laserlichtquelle (4) unmittelbar aufeinanderfolgenden Bildeinhalte voneinander subtrahiert werden, so dass die übrigen Bildinhalte in ihrer Intensität herabgesetzt und die Lasermarkierung demgegenüber zur Auswertung hervorgehoben ist.

6. Bildverarbeitungssystem nach Anspruch 1, **dadurch gekennzeichnet, dass** zur Objektbeleuchtung vorgesehene Leuchtmittel (11) bei der Erfassung der Lasermarkierung ausgeschaltet oder in ihrer Intensität vermindert sind.

7. Bildverarbeitungssystem nach Anspruch 1, **dadurch gekennzeichnet, dass** bei Anwendung zum Form- bzw. Positionsabgleich eines in ein größeres Werkstück bzw. einen Patientenkörper einzubringenden Elements bzw. Implantats oder sonstigen Hilfsmittels, wobei das Abbild des Arbeits- bzw. Operationsbereichs, in den das Element, Implantat oder sonstiges Hilfsmittel eingebracht wird, der von der Kamera aufgenommen Bildinhalt auf denselben Maßstab eingestellt ist wie ein Abbild des Elements, Implantats oder sonstigen Hilfsmittels, welches in das Abbild des Arbeits- bzw. Operationsbereich, insbesondere verschieb- und/oder rotierbar, einblendbar ist.

8. Bildverarbeitungssystem nach Anspruch 1, **dadurch gekennzeichnet, dass** eine Auflagefläche zum unmittelbaren Aufbringen des Elements bzw. Implantats oder Hilfsmittels auf der im wesentlichen horizontal gerichteten Oberfläche des Bildschirms (3) vorgesehen ist zwecks Größenabgleichs der Bilddarstellung mindestens eines Teils des Arbeitsbereichs im Maßstab 1:1 mit einem Original des Elements bzw. Implantats oder Hilfsmittels, wobei der Bildschirm selbst oder eine darauf befestigte Schutzscheibe als Auflagefläche dient und dabei unmittelbar eine Arbeitsoberfläche bildet.

9. Bildverarbeitungssystem nach Anspruch 1, **dadurch gekennzeichnet, dass** Mittel zum maßstabsgerechten Überlagern (22) einer das Element bzw. Implantat oder Hilfsmittel betreffenden Bildinformation in dem Speicher mit einem Abbild des Arbeits- bzw. Operationsbereichs vorgesehen sind, wobei das Abbild des Arbeits- bzw. Operationsbereichs und die das Element bzw. Implantat betreffende Bildinformation untereinander denselben Maßstab aufweisen und dass Verschiebe- oder Rotationsmittel (26) vorgesehen sind, mittels denen das Abbild des einzubringenden Elements bzw. Implantats oder Hilfsmittels auf dem Bildschirm (3) in seiner Position im Hinblick auf das Werkstück in der Ebene der Darstellung verschieb- oder drehbar ist, um dessen geometrisches Zusammenwirken mit dem Werkstück bzw. dem Körper des Patienten im Arbeits- bzw. Operationsbereich zu optimieren.

10. Bildverarbeitungssystem nach Anspruch 9, **dadurch gekennzeichnet, dass** den Bildspeichermitteln nachgeschaltete Mittel zur hervorhebenden Auswahl (16) von Teilelementen des Bildes vorgesehen sind, wobei die Auswahl jeweils nach einem vorgegebenen Kriterium aufgrund der aufgenommenen Bildinformation erfolgt und die hervorhebend ausgewählten Teilelemente zu einer schematisierten Bildinformation als inhaltsbasierten Bildbeschreibung aufgrund visueller Merkmale zusammengefasst sind.

11. Bildverarbeitungssystem nach Anspruch 10, **dadurch gekennzeichnet, dass** die Form und/oder Abmessungen sowie visuelle Merkmale des Bereichs der ausgewählten Bildelemente zusammengefasst ein Auswahlkriterium für ein in den Arbeits- bzw. Operationsbereich einzubringendes Element bzw. Implantat oder Hilfsmittels in einem nachgeschalteten nach Art eines Katalogs organisierten Speicher (20) bilden, in dem diese auch als zu einem inhaltsbasierten Vergleich geeigneten Abbildungen oder entsprechende Formmerkmalskennzeichnungen gespeichert sind.

12. Bildverarbeitungssystem nach Anspruch 11, **dadurch gekennzeichnet, dass** Mittel (18) für eine Formselektion aufgrund eines Größen- und/ oder Formähnlichkeitskriteriums vorgesehen sind, um die Ausgabe der Form und/oder des Typs eines Elements bzw. Implantats oder Hilfsmittels aufgrund des Größen- und/oder Ähnlichkeitskriteriums in einem nach Art eines Katalogs organisierten Speicher (20) zur Ausgabe als Bild- und oder Typenbezeichnungsinformation zu adressieren,.

13. Bildverarbeitungssystem nach Anspruch 1, **dadurch gekennzeichnet, dass** die Laserlichtquelle (44) derart angebracht ist, dass das Laserstrahlenbündel mindestens für einen Teil von dessen Länge durch den Arbeitskanal eines starren Endoskops (41) bzw. Laparoskops oder sonstigen vergleichbaren Geräts hindurchgeführt ist.

14. Bildverarbeitungssystem nach Anspruch 1, **dadurch gekennzeichnet, dass** der Bildschirm ein (51) separates Bauteil bildet, eine autarke Stromversorgung aufweist und über eine Funkschnittstelle mit dem übrigen System verbunden ist.

## Claims

1. An image processing system for the reproduction of an image content in a predetermined scale comprising:
- an optical camera with a large depth of field, a digital image sensor, a laser light source generating an information representative of a geometric distance in the form of a pattern, which is detected and processed with the image data captured by the camera,
- image memory means (9) and a monitor (3) for reproducing the captured image data, wherein
- the camera (1) having a separator (8) downstream connected thereto which separate the information representative of a geometric distance from the rest of the image data,
- the separator (8), having an evaluator (13) downstream connected thereto which determine and output physical distance data from the information representative for geometric distance,
- an actuator (14) for varying the size of the reproduced image on the monitor (3) in the manner of a digital zoom, controlled by the geometric distance data as an input variable, for adjusting the size of the image representation on the monitor in accordance with a scale factor, the physical distance data being included as a multiplier, such that an object contained in the picture data having a physical size corresponding to the physical distance data it is reproduced on the monitor in a size, which is defined by the preselected scale factor.

2. The system of claim 1, wherein the information representative of a geometric distance within the image captured by the camera (1) is at least one laser marking generated by a beam from the laser source (4) directed in a defined direction with respect to the optical axis of the camera (1), wherein the distance between two laser markings or the distance between a laser marking and the optical axis of the camera (1) is the information representative of a geometric distance.

3. The system of claim 1, wherein means are provided for generating several laser markings arranged around the optical axis of the camera (1) in symmetric positions, and wherein the image processing system further inhibits those laser markings from further processing within the data of the image representation, that are detected in a position differing from the symmetry position with respect to the optical axis of the camera by more than a predetermined amount

4. The system of claim 1, wherein for an object plane arranged in a non-orthogonal direction with respect to the optical axis of the camera, wherein the image processing system adjusts the image representation on the monitor with a locally variable scale factor-corresponding to a trapezoidal or keystone correction-such that the resulting locally relevant scale factors for the image representation are interpolated between the positions of at least two known scale factors derived from the respective data representative for a geometric distance and are accordingly extrapolated outside the known scale factors.

5. The system of claim 1, wherein the separator (8) is provided for determining the difference of the data of two images representations following each other in immediate succession during an on and an off state of the laser light source (4), to highlight the laser markings for further evaluation while the rest of the image representation is reduced in its intensity.

6. The system of claim 1, wherein means are provided for switching off or reducing the intensity of lightings means (11) for the object illumination during the detection of the laser markings.

7. The system of claim 1, wherein a comparator is provided for a form or size comparison of an element or implant to be introduced into a working or surgical field respectively with the camera provided image representation of the work or surgical field in which the element or implant is to be inserted is set to the same scale as the image representation of the element or implant, which can be faded, in particular moved and/or scaled within the image representation of the work or surgical field.

8. The system of claim 1, wherein for a form or size comparison, of a physical element or a physical implant to be introduced into a working or a surgical field respectively with the image representation in the original scale of the working or surgical field in which the element or implant is to be inserted a monitor display is provided having a supporting surface extending substantially horizontally for direct physical contact with the element or implant.

9. The system of claim 1, wherein storage means are provided for the scaled superimposing (22) of an image representation of an element or an implant to be inserted into a working or a surgical field respectively with an image representation of the working or surgical field, whereby the image representation of the working or surgical field and image representation of the element or implant are in the same scale, and further shifting or rotation means are provided (26) by means of which the image representation of the element or implant may be displaced in its position on the monitor display (3) in its position with respect to the working or surgical in order to improve its geometric interaction with the working or surgical field respectively.

10. The system of claim 9, wherein downstream connected to said storage means for image representation are a form discriminator (16) are provided by partial image representation, wherein the selection is made according to a predetermined criterion from the recorded image representation data and highlighting selected elements into an image representation data summarized as content-based image representation description based on a selection of elements basing on visual features.

11. The system of claim 10, wherein the shape and/or dimensions and visual characteristics of the range of the selected picture elements summarized a selection criterion for the working or operation range to be introduced element or implant or aid in a downstream manner of a catalogue organized memory (20) form in which they are stored as a content-based comparison illustrations suitable or appropriate shape feature identifications.

12. The system of claim 11, wherein a comparator (18) form the basis of a size and/or shape similarity criterion are provided to the output of the shape and/or the type of an element or implants or resource due to the size and/or similarity criterion in an organized way to store a catalogue (20) for outputting an image representation, or to address planning and data-type designation.

13. The system of claim 1, wherein the laser light source (44) is mounted in such way that the laser beam passes at least for part of its length through the working channel of a rigid endoscopes (41), laparoscope or similar device

14. The system of claim 1, wherein the monitor (51) is formed as a separate component, comprising an independent power supply having a data connection via a radio interface to the rest of the system.

## Revendications

1. Un système de traitement d'image, pour la reproduction d'un contenu d'image à une échelle préétablie composé de :
- une caméra optique avec une grande profondeur de champ, un capteur d'image numérique, une source de lumière laser produisant une information représentative d'une distance géométrique sous la forme d'un modèle, qui est détecté et traité avec les données de l'image capturées par la caméra,
- des moyens de mémoire d'image (9) et un écran (3) pour reproduire les données de l'image capturée, dans lesquels
- une caméra (1) avec un séparateur (8) connecté en aval, qui sépare l'information représentative d'une distance géométrique du reste de l'image,
- le séparateur (8), avec un évaluateur (13) connecté en aval, qui détermine et exporte des données de distance physique à partir de l'information sur la distance géométrique,
- un actionneur (14) pour faire varier la taille de l'image reproduite sur l'écran (3) à la manière d'un zoom numérique, contrôlé par les données de distance géométrique comme une variable d'entrée, permettant d'ajuster la taille de la représentation de l'image sur l'écran conformément à un facteur d'échelle, les données de distance physique étant comprises comme un multiplicateur, telle qu'un objet contenu dans l'image dont la taille physique de données correspondant aux données distance physique c'est reproduit sur l'écran, avec une taille qui est définie par le facteur d'échelle présélectionné.

2. Le système selon la revendication 1, dans lequel le représentant de l'information de distance géométrique dans l'image capturée par la caméra (1) est au moins un laser de marquage généré par un faisceau de la source laser (4) dirigé dans une direction définie par rapport à l'axe optique de la caméra (1), dans lequel la distance entre deux marquages laser ou la distance entre un marquage laser et l'axe optique de la caméra (1) est le représentant de l'information d'un distance géométrique.

3. Le système selon la revendication 1, où les moyens sont fournis pour générer plusieurs marquages laser disposées autour de l'axe optique de la caméra (1) dans des positions symétriques, et dans laquelle le système de traitement d'image plus inhibe ces marquages laser de poursuite de la procédure au sein des données de la représentation de l'image, qui sont détectées dans une position différente de la position de la symétrie par rapport à l'axe optique de la caméra par plus d'un montant prédéterminé.

4. Le système selon la revendication 1, dans lequel un avion objet disposé dans une direction non orthogonale par rapport à l'axe optique de la caméra, dans lequel le système de traitement d'image permet d'ajuster la représentation de l'image sur l'écran avec un facteur d'échelle localement variable - correspondant à une correction trapézoïdale ou clé de voûte - tel que les facteurs d'échelle localement pertinents qui en résulte pour la représentation de l'image sont interpolés entre les positions d'au moins deux facteurs d'échelle connu tirés des données respectives représentant une distance géométrique et sont par conséquent extrapolés à l'extérieur les facteurs d'échelle connu.

5. Le système selon la revendication 1, dans lequel le séparateur (8) est fourni pour la détermination de la différence des données de représentation de deux images suivantes immédiate pendant un état allumé et un état d'arrêt de la source de lumière laser (4), pour mettre en surbrillance le marquage laser pour une évaluation approfondie, tandis que le reste de la représentation de l'image est réduit dans son intensité.

6. Le système selon la revendication 1, où les moyens sont fournis pour éteindre ou diminuer l'intensité des moyens d'éclairages (11) pour l'éclairage de l'objet au cours de la détection des marquages laser.

7. Le système selon la revendication 1, dans lequel un comparateur est fourni pour une comparaison de forme ou la taille d'un élément ou un implant destinés à être introduits dans un espace de travail ou un champ opératoire, respectivement avec la représentation de l'image du champ opératoire dans lequel l'élément ou l'implant doit être inséré est sur la même échelle que la représentation de l'image de l'élément ou l'implant, qui peut être inséré, en particulier déplacé et / ou tourné dans la représentation d'image de l'espace de travail ou du champ opératoire.

8. Le système selon la revendication 1, dans lequel un formulaire ou taille comparaison d'un élément physique ou un implant physique destinés à être introduits dans un espace de travail ou un champ opératoire respectivement avec la représentation de l'image dans l'échelle originale de l'espace de travail ou du champ opératoire dans lequel l'élément ou l'implant doit être inséré un écran d'affichage est fourni en ayant un support surface s'étendant presque horizontalement pour un contact physique direct avec l'élément ou l'implant.

9. Le système selon la revendication 1, dans lequel un stockage moyen fourni pour la mise à l'échelle superposé (22) d'une représentation de l'image d'un élément ou un implant à insérer dans un travail ou un champ opératoire respectivement avec une représentation de l'image de l'espace de travail ou opératoire, par lequel la représentation de l'image de l'espace de travail ou opératoire et représentation de l'image de l'élément ou l'implant se trouvent dans la même échelle, et encore déplacement ou rotation, sont fournis (26) par moyen dont la représentation de l'image de l'élément ou l'implant peut être déplacée de sa position sur l'écran d'affichage (3) dans sa position en ce qui concerne le travail ou chirurgical afin d'améliorer son interaction géométrique avec le domaine fonctionnel ou chirurgical respectivement.

10. Le système selon la revendication 9, dans lequel des moyens de rangement pour la représentation de l'image connecté en aval à sont un discriminant de la forme (16) sont fournis par la représentation de la partie de l'image, où la sélection est faite selon un critère prédéterminé d'après les données de représentation de l'image enregistrée et mise en évidence des éléments sélectionnés dans une représentation d'image de données résumées comme description de représentation basé sur le contenu image basée sur une sélection d'éléments en se basant sur les caractéristiques visuelles touchant.

11. Le système selon la revendication 10, dans lequel la forme et/ou les dimensions et les caractéristiques visuelles de la gamme des éléments image sélectionné résume un critère de sélection pour le travail ou l'opération rang à élément introduit ou implant ou à l'aide d'une manière en aval d'une forme de mémoire (20) catalogue organisé dans lequel ils sont stockés sous une identification de fonctionnalité comparaison basée sur le contenu illustrations forme convenable ou approprié.

12. Le système selon la revendication 11, dans lequel un comparateur (18) constitue la base d'un critère de similarité taille et/ou forme fournis à la sortie de la forme et/ou le type d'un élément ou d'implants ou de ressource en raison du critère de taille ou de la similitude de manière organisée pour stocker un catalogue (20) pour produire une représentation de l'image, ou la désignation de planification et de type de données d'adresse.

13. Le système selon la revendication 1, dans lequel la source de lumière laser (44) est montée de telle manière que le faisceau laser passe au moins pendant une partie de sa longueur par le canal d'un endoscope rigide (41), laparoscope ou un appareil similaire.

14. Le système selon la revendication 1, dans lequel l'écran (51) est formé en un composant distinct, comprenant une alimentation indépendante ayant une connexion de données via une interface radio pour le reste du système.
